# EUROPEAN PATENT SPECIFICATION

(11) **EP 1 901 870 B1**
(45) Date of publication and mention of the grant of the patent: **17.06.2009**
(21) Application number: 06758706.3
(22) Date of filing: 26.04.2006
(51) Int. Cl.: B22F 1/00, B22F 9/16, B32B 3/26

(54) **EMBOSSED METALLIC FLAKES PROCESS AND PRODUCT**
VERFAHREN ZUR HERSTELLUNG VON GEPRÄGTEN METALLFLOCKEN UND PRODUKT
PROCEDE DE FABRICATION DE FLOCONS METALLIQUES CANNELES, ET PRODUIT CORRESPONDANT

(30) Priority: 26.04.2005 US 674808 P
(43) Date of publication of application: 26.03.2008
(73) Proprietor: Avery Dennison Corporation, Pasadena, CA 91103 (US)
(72) Inventor: RETTKER, James, P., Crown Point, Indiana 46307 (US)
(74) Representative: Hock, Joachim
(86) International application number: PCT/US2006/016115
(87) International publication number: WO 2006/116641

(56) References cited:
- WO-A-03/046245
- US-A- 4 116 710
- US-A- 5 672 410

## Description

### FIELD OF THE INVENTION

This invention relates to a process for producing embossed metal flakes and the use of such of flakes in coatings and printing inks. More particularly, the process involves techniques for producing embossed flakes having high levels of brightness and color intensity when formulated in coatings and printing inks.

### BACKGROUND

Metallic flakes have been used for many years in decorative coatings to produce different visual effects. Metallic flakes are used in metallic automotive paints, for example. These flakes are typically made by vacuum metalizing the smooth surface of a release coat applied to a flexible temporary carrier film, solubilizing the metalized release surface to remove the metal film from the carrier, and breaking up the metal into flakes.

In addition to automotive paints, metallic flakes have been used in other coating compositions, paints, enamels, lacquers, and the like, including coatings that produce a highly reflective metalized surface for metallic-like or mirror-like optical effects. In these coatings, small particle size metal flakes below about 50 microns in size can produce good reflectivity along with the opacity necessary to provide complete 100% coverage for the mirror-like effects. Larger flakes which may be reflective are usually more spread out when applied as a coating, and therefore, may not produce the necessary opacity or hiding ability for yielding a highly reflective mirror-like surface.

The small metal flakes also have tended to be more useful in compositions such as printing inks where the larger flake sizes are not as usable in certain types of printing equipment.

In another development, metallic flakes have been produced with embossed patterns in the form of diffraction grating or holographic image patterns. These flakes produce certain iridescent effects when used in coatings or printing inks. These flakes have been made by a process described in U.S. Patent 5,672,410 to Miekka et al., assigned to Avery Dennison Corporation. In the process of making embossed flakes according to the '410 patent, metallic flakes having a controlled particle size below about 50 microns are produced. The metallic flakes can be produced by different embossing techniques followed by metalizing the embossed surface, stripping the metal to form a dispersion of flakes, and then breaking up the metal flakes into smaller size flakes approximately 10 to 50 microns in size. The dispersed metal particles are subjected to high speed mixing or ultrasonic mixing which breaks up the particles into the desired size range without destroying the reflectivity of the flakes. The metallic film obtained by this process resembles the brilliance, reflective gloss and hiding power of commercial metallic foils. Due to the natural orientation of the single layer leafing flake, even when embossed, small amounts of pigment will cover a very large surface area.

US 4,116,710 discloses a method comprising providing a substrate with a coating on at least a portion of the substrate, the coating consisting essentially of an about 200 to about 600 angstrom thick vapor deposited metal layer, dissolving the substrate to separate at least a portion of the coating from the substrate to provide a plurality of generally separate irregularly shaped metal platelets with a largest dimension up to about 5000 microns, and introducing the metal platelets into a carrier suitable for use in a coating composition, wherein the metal coating is preferably aluminum.

### SUMMARY OF THE INVENTION

Briefly, one embodiment of the present invention comprises the process defined by claim 1.

Another embodiment of the invention comprises reflective metal flakes defined by claim 5.

The process of this invention controls the color intensity or chromaticity and brightness of embossed flakes and produces flakes of large particle size with high levels of color intensity and brilliance. The embossed flakes of this invention have application to coatings and printing inks that produce extremely high brightness characterized as an optically apparent glitter or sparkle effect in combination with high color intensity or chromaticity. The embossed flakes also can be used to produce similar optical effects when used in the decorative layers of multi-layer laminates, including those subjected to thermoforming.

### BRIEF DESCRIPTION OF THE DRAWINGS

FIG. 1 is a schematic cross sectional view illustrating an intermediate embossing step in a process according to principles of this invention.
FIG. 2 is a schematic diagram illustrating diffraction grating embossments formed at a 45° angle.
FIG. 3 is a schematic diagram illustrating diffraction grating embossments formed at a 60° angle.
FIG. 4 is a schematic diagram illustrating reflection angles used for measuring color intensity and chromaticity with a multi-angle spectrophotometer.

### DETAILED DESCRIPTION

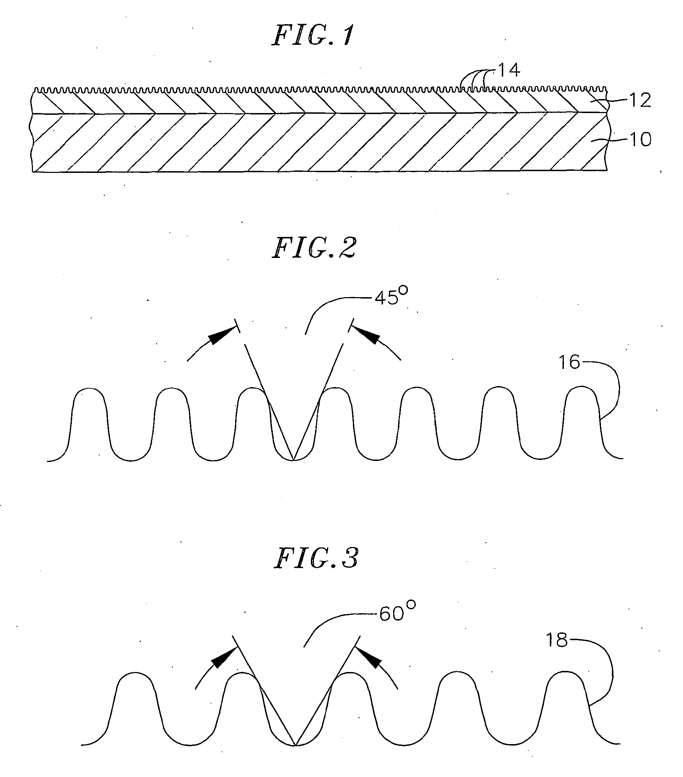

Referring to FIG. 1, a temporary carrier film 10 passes into a coater for applying a release coat 12 to at least one side of the carrier film. In a preferred embodiment, both sides of the carrier film are coated with a thin film of the release coat material. FIG. 1 illustrates one embodiment of the invention which includes embossing the release coat to form a pattern of embossments 14 as described below. The embossed carrier is then metalized with a thin reflective metal film as described below.

The carrier film 10 is preferably a flexible, foldable, heat-resistant polymeric casting film, preferably biaxially oriented PET. The polyester films Mylar or Hostaphan from American Hoechst are examples of the preferred casting films. The preferred carrier film has an extremely smooth casting surface substantially in the absence of adherent fine particulate materials such as filler particles commonly used for roughening the surface to improve slip properties. The carrier film used for this invention preferably has no slip additive. Such polyester film is commercially available in different grades. A PET Hostaphan grade of 3000 or better, based on surface roughness measurements, is useful in this invention.

The carrier film can be embossed by various techniques, including embossing the release coat applied to the carrier or embossing the metal layer of a metalized carrier film. The above-mentioned '410 patent describes four separate methods which may be used for forming the embossment pattern but do not form part of the present invention. The preferred embossing process involves applying a release coat to the carrier and embossing the release coat as follows.

The release coat material 12 comprises various polymeric materials which can be embossed with accurately formed embossments (described below) and which can be easily solubilized in an organic solvent. Examples of suitable release coat materials include acrylic resins such as PMMA, acrylic copolymers, PVC, and polystyrene. The release coat material is solubilized in a suitable organic solvent and applied to the carrier film by roll coating techniques. The coated carrier is then subjected to heat for drying the release coat to produce an extremely smooth release surface. The preferred thickness of the release coat is produced by applying the release coat at about 0.8 grams per square meter per side of the carrier, or 1.6 grams per square meter total.

The release surface is embossed with an embossing roll to form the pattern of embossments 14 on the release surface. The embossments are preferably in the form of a diffraction grating pattern formed by embossing closely spaced apart and regularly spaced apart parallel grooves in the release surface. In one embodiment, the diffraction grating is formed by a regular pattern from 5,000 to less than 14,000 groves per centimeter. In another embodiment, the diffraction grating structure is from 10,000 to 12,500 grooves per centimeter.

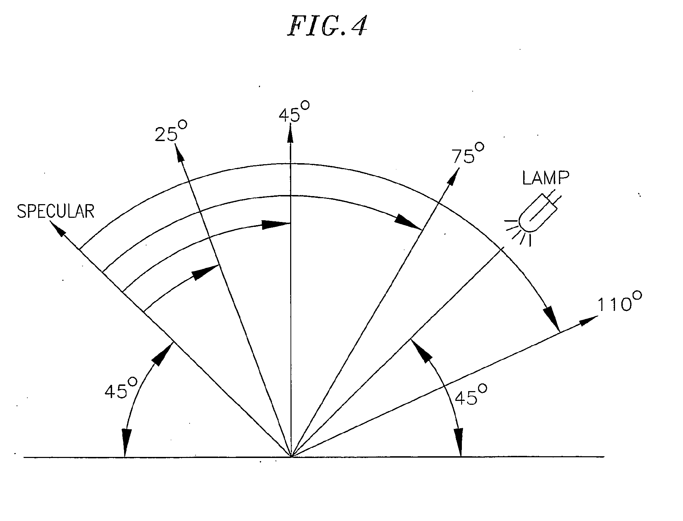

Prior to embossing, the release coat is allowed to dry or solidify. The embossing step is then carried out by heating the release coat to above its softening temperature and then embossing the diffraction grating pattern in the release surface. The embossing roll preferably forms the embossments in a monoruled pattern -- straight parallel grooves identically shaped and uniformly spaced apart in a single embossing path across the release surface. In one embodiment, the groove pattern has a wavy or sinusoidal cross-sectional structure such as that shown in FIG. 4.

The grating structure from which the flakes of this invention are made can have a groove depth from 125 nm to 140 nm, and more preferably, from 130 nm to 135 nm.

The diffraction grating pattern of embossments described in the '410 patent have been produced commercially in the past by monoruling at a 45° angle. FIG. 2 illustrates such a monoruled embossment pattern 16 and the 45° ruling angle defined as a line drawn from the base of the groove tangential to the adjacent top portion of the groove.

According to the present invention, the embossing roll forms a diffraction grating pattern by monoruling identically shaped and uniformly spaced-apart parallel grooves each having an embossment angle greater than 45°. In one embodiment, the embossment pattern is a monoruled 60° angle diffraction grating pattern 18 as shown in FIG. 3.

After forming the embossed release surface, the embossed carrier is passed through a vacuum metalizer for vacuum depositing a metal film on the embossed release coating. In one embodiment in which the release coat is coated on both sides of the carrier, both sides are embossed, and the metal film is vacuum deposited on both sides of the carrier. The thickness of the deposited metal monolayer film is from about 50 to 1,000 angstroms (5-100 nm), controlled by the speed of the web and the evaporation rate. Suitable bright metals for deposition include aluminum, chromium, copper, indium, steel, silver, gold, nickel or Nichrome. Aluminum is a presently preferred metal film.

In one embodiment, a preferred metal thickness range for a single layer metalized aluminum flake product is from about 50 nm to about 100 nm. The desired metal thickness also can be from 1.0 to 3.5 optical density. Optical density is measured on a MacBeth TR 927 densitometer.

The metal coated embossed carrier is then passed through a metal stripping machine for removing the metal from the carrier to form flakes. Preferably, the metalized carrier is passed around a series of rollers in a tank containing a suitable solvent for solubilizing the release coat. The preferred solvent is acetone. The metal film passes over the rollers and then past a series of doctor blades for removing the metal particles and release coat material from the carrier. The dispersion of flakes and release coat polymer in the solvent is then pumped to a slurry tank. The resultant dispersion in the slurry tank has a percent solids weight from 2% to 4% based on the aluminum flakes and residual polymer solids dispersed in the solvent.

The flakes contained in the resulting slurry have a desired average particle size at or above 75 microns. These flakes are maintained at a particle size at or above 75 microns by omitting any high energy mixing or particle sizing steps following the metal stripping process. High energy particle sizing such as centrifuging, sonolater treatment or high shear mixing are avoided. Possibly low shear mixing may be suitable in some instances, but with the object of controlling particle size to at or above 75 microns. High speed mixing, in addition to reducing particle size, can reduce flake brightness. In one embodiment, single layer aluminum flakes produced by this process had an average (D50) particle size above 75 microns. Flakes within a 75 to 200 micron size range can be produced at optical densities within the range of about 1.0 to about 3.5. In one embodiment, the range of desired particle sizes is generally from about 75 to about 150 microns to produce certain optical effects described below. Such particles have been produced within this size range at an optical density of about 2.0 and by following the process steps described above. Larger particle sizes can be produced with thicker flakes, say greater than about 3.5 optical density.

The present invention also can produce embossed flakes greater than 200 microns in particle size. In one embodiment flakes with a particle size above 200 microns were produced from thicker flakes having an optical density of 3.0 and above.

Particle size measurements as described herein are made using a Horiba LA 910 instrument.

Single layer metal flakes having an average D50 particle size greater than 75 microns have been produced using a diffraction grating similar to FIG. 4, to produce flakes having a thickness in the range of 50 nm to 100 nm. In one embodiment, the flake thickness was about 90 nm. These embossed metal flakes have been produced with a diffractive grating pitch of less than 14,000 lines per centimeter. These diffractive flakes, in one embodiment, had a pitch of less than 12,500 lines per centimeter, and in another embodiment, the diffractive pattern was in the range from about 10,900 to less than 12,000 lines per centimeter. These embossed metal flakes had a groove depth of less than about 140 nm, and in one embodiment, groove depth measured from about 130 nm to about 135 nm. These embossed flakes were characterized by a desired groove depth to flake thickness ratio of greater than 1.0.

The large embossed flakes produced by this invention can be used in various types of coatings to produce certain controlled optical effects. In one embodiment coatings having greater color intensity combined with a glitter or sparkle effect can be produced. Embossed particles at about 100 micron size can just start to be seen by non-magnified visual observation, which reveals the glitter or sparkle effect produced by the visually observable embossments.

In one embodiment, the embossed flakes embossed at an angle greater than 45°, and 60° in particular, produced greater reflectivity or brilliance and more color intensity in a coating when compared with prior art flakes made with embossing at a 45° angle.

The glitter or sparkle effect is produced by the larger particles embossed by the techniques of this invention which produce greater reflectivity than the reflectivity produced by the smaller flakes, say about 50 microns average particle size. This comparison is between the larger particles of this invention made by replicating embossments greater than 45°, and in one embodiment, at a 60° angle, when compared with smaller 50 micron flakes made by replicating embossments at a 45° angle. The smaller flakes have lower reflectivity, i.e., are not as mirror-like or are less brilliant. The increased reflectivity of the larger flakes is produced across all colors of the color spectrum.

Flakes made with embossments at angles greater than 45° can appear brighter than the same size flake made with embossments at 45°. Flakes made with 60° embossments have been observed to have greater brightness in fluorescent light. The flake made with embossments greater than 45° also had visually observable greater color intensity and color shift. The greater area of the replicated embossments available for reflecting incident light is considered to be a reason for the greater brightness and color effects.

As mentioned, the glitter or sparkle effect is produced in combination with a greater color intensity when compared with the smaller 50 micron flakes; and the greater color intensity combined with the glitter effect has been observed visually from various coatings as well as demonstrated by numerical data produced by color measurements taken by a multi-angle spectrophotometer as shown in the examples below.

The larger flakes of this invention can be used to produce the described optical effects in various coating compositions such as paints, inks, enamels and print coats. Resinous binders useful with the invention include acrylic and nitrocellulosic resins. One application of the invention comprises a nail polish enamel containing embossed flakes greater than 75 microns, which exhibits a brighter glitter or sparkle effect and greater color intensity or color shift at certain observation angles when compared with a nail polish enamel containing the smaller (50 micron) flakes. This application of the invention has been observed using a nitrocellulose enamel such as that described in International Patent Publication WO 02/03913 to Kirker Enterprises, Inc., This nail polish enamel was drawndown on a card, dried and observed for its optical effects. The application of the larger particle size embossed flake with the extremely thin angstrom level particle thickness produces the glitter or color shift effects in coatings such as enamels with the embossed flake sizes at 75 microns or more. The individual particles can just be seen by the naked eye at 100 microns sufficient to observe the glitter or rainbow effects of the embossed particles. The ability of these larger embossed flakes to lay down flat in various coatings also enhances the reflected light and color shift effects which are visually observable.

Other uses of the larger embossed flakes of this invention are in colored printing inks, inks used in silk screen processes, and cosmetic formulations. The larger flakes of this invention can add glitter or sparkle effects to printing inks and dyes.

In one embodiment, the larger flakes of this invention were found to have good orientation in nitrocellulosic coating compositions which can be useful for printing inks as well as nail polish enamel.

Another application is for producing certain visual effects in print coats used in various multi-layer laminates. These can include laminates having a thermoformable polymeric substrate base layer, an opaque pigmented base coat or paint coat applied to the substrate, a metallic print coat applied to the pigmented base coat by various printing techniques for producing a decorative print pattern, and an optional outer clear coat that can be a protective weatherable and abrasion-resistant clear coat. The resulting laminate can be thermoformed to form various shapes without degrading the reflective appearance of the metallic print coat.

The larger metallic flakes also can be used in highly reflective metallic layers contained in similar thermo-formable multi-layer laminates.

### EXAMPLES

Embossed flakes greater than 75 microns in size were produced according to the previous description. A PET carrier was coated with an acrylic release coat, embossed with a diffraction grating pattern having a 60° monoruled embossment pattern as described previously, metalized with a vapor deposited aluminum film, and stripped to form a metal flake dispersion. Layer thickness for the metalized film in these examples was approximately 2.0 optical density. The flakes were removed directly after stripping for testing. Particle-sizing such as centrifuging or sonic mixing, that would otherwise reduce particle size, were avoided. The act of centrifuging may not reduce particle size, but running through a pump or high speed mixing will reduce the particle size. The embossed particles had a D50 average particle size of about 114 microns. A sample was drawndown on the black side of a leneta card. This sample was compared with a similar sample containing 50 micron flakes made by 45° embossing also drawndown on the black side of a leneta card. The larger flakes were visually observed to display a more pronounced color shift and color intensity than the smaller flakes.

The following test data were taken from color measurements using an X-Rite MA 58 Multi-Angle SpectroPhotometer. Color values were taken at three angles of measurement, 45°, 75° and 110°, as shown in FIG. 4. The color readings with higher numerical values indicate greater color intensity. Color readings measured color intensity as follows:
a* (positive) = red
a* (negative) = green
b* (positive) = yellow
b* (negative) = blue
C* = a*(squared)+b*(squared) = a summation of all averages, measuring chromaticity or color intensity

### Example 1

| Large particle size flakes | | |
|---|---|---|
| 45°: a*=-6.51 | b*=4.54 | C*=7.94 |
| 75°: a*=21.94 | b*=-37.21 | C*=43.20 |
| 110°: a*= 5.53 | b*=29.11 | C*=29.63 |

| Smaller particle size flakes | | |
|---|---|---|
| 45°: a*=-15.14 | b*=-7.67 | C*=16.97 |
| 75°: a*=19.82 | b*=-33.22 | C*=38.68 |
| 110°: a*=5.19 | b*=15.44 | C*=16.29 |

These test data showed that the larger flakes had better color intensity, especially at 75° and 110°, than the smaller flakes even though the larger flakes were more spread out with a greater amount of space between particles than the smaller flakes.

### Example 2

The larger particle size flakes of Example 1 were decanted by letting the flakes settle to the bottom of a vessel and removing the resin-rich liquid layer from the top of the vessel. The clear liquid was at 2.4% resin solids (when measured by drying and reporting weight difference). The decanted sample contained 4.1% solids by weight. The test data showed that the flakes were brighter with more intense color than the sample that was not decanted.

| 2.4% solids - no decant | | |
|---|---|---|
| 45°: a*=-13.36 | b*=-1.03 | C*=13.40 |
| 75°: a*=31.55 | b*=-51.69 | C*=60.55 |
| 110°: a*= 5.73 | b*=24.64 | C*=25.30 |

| 4.1% solids - decanted | | |
|---|---|---|
| 45°: a*=-28.85 | b*=12.59 | C*=31.48 |
| 75°: a*=40.72 | b*=-60.63 | C*=73.03 |
| 110°: a*=2.34 | b*=37.20 | C*=37.27 |

### Example 3

The larger particle size flakes of Example 1 were added to the 50 micron flakes (10 parts 50 micron flakes to 2 parts 100 micron flakes) contained in a lacquer and drawndown on a leneta card, both sides. These samples were compared with a similar drawdown of the 50 micron flakes of Example 1. The results showed improved color intensity with the addition of the larger flakes.

| 50 micron flakes only (black) | | |
|---|---|---|
| 45°: a*=-40.29 | b*=-1.00 | C*=40.30 |
| 75°: a*=51.38 | b*=-80.13 | C*=95.19 |
| 110°: a*= 5.33 | b*=43.79 | C*=44.11 |

| 114 micron flakes added (black) | | |
|---|---|---|
| 45°: a*=41.45 | b*=-1.52 | C*=41.47 |
| 75°: a*=52.91 | b*=-81.40 | C*=97.09 |
| 110°: a*= 4.10 | b*=42.71 | C*=42.91 |

| 50 micron flakes only (white) | | |
|---|---|---|
| 45°: a*=-38.82 | b*= 1.97 | C*=38.87 |
| 75°: a*=42.04 | b*=70.80 | C*=82.34 |
| 110°: a*= 5.06 | b*=34.50 | C*=34.87 |

| 114 micron flakes added (white) | | |
|---|---|---|
| 45°: a*=4.6.17 | b*= 2.47 | C*=46.24 |
| 75°: a*=4.5.06 | b*=-73.90 | C*=86.59 |
| 110°: a*= 4.91 | b*=34.17 | C*=34.52 |

## Claims

1. A process for making embossed fine particulate thin metallic flakes having brightness and color intensity, comprising providing a release surface (12) on a carrier (10), embossing the release surface (12) with a diffraction grating pattern (14,16,18) having an angular ruling pattern greater than 45°, metalizing the embossed release surface (12) with a thin reflective metal film, removing the metal film from the release surface (12) to form a solvent dispersion of embossed metal flakes that have replicated the diffraction grating pattern, and controlling the particle size of the flakes contained in the dispersion to maintain the embossed flakes contained therein at a D50 particle size at or above 75 microns.

2. The process of forming a first coating containing the embossed flakes of claim 1 dispersed in a polymeric binder, in which the first coating has a higher chromaticity reading and a higher color intensity reading at 75° and 110° angular measurements when measured on a multi-angle spectrophotometer, when compared with a second coating containing a dispersion of D50, 50 micron size embossed flakes made by a similar process and contained in the same polymeric binder.

3. The process according to claim 1 in which the embossed flakes have a diffraction grating pattern of less than 14,000 grooves per centimeter, a flake thickness from 50 nm to 100 nm, and a groove depth from 125 nm to 140 nm.

4. The process according to claim 1 in which the metallic flakes contained in the solvent dispersion are subjected to no applied energy that would reduce particle size greater than low speed mixing, or would reduce particle size more than 20 microns.

5. Reflective metal flakes which have been embossed by replicating a diffraction grating pattern having a monoruled embossing angle above 45°, the particles having a D50 average particle size at or above 75 microns, and a flake thickness from 50 nm to 100 nm.

6. The flakes according to claim 5 in which the embossed flakes have a particle size range of (a) or (b):
(a) from 75 to 200 microns, or
(b) from 75 to 150 microns; and
in which the metallic flakes have a thickness range of (c) or (d):
(c) from 5 nm to 100 nm, or
(d) from 50 nm to 100 nm; or alternatively, an optical density from 1.0 to 3.5.

7. The flakes of claim 5 in which the diffraction grating pattern has from 5,000 to less than 14,000 grooves per cm.

8. The flakes of claim 5 in which the embossed flakes are contained in a dry film coating having a greater measured chromaticity and color intensity at 75° and 110° (measured via a multi-angle spectrophotometer) when compared with a similar coating containing flakes embossed at a diffraction grating pattern at 45° and having a particle size of 50 microns.

9. The flakes of claim 5 in which the embossed flakes have an optical density of 3.0 or more and a D50 average flake size greater than 200 microns.

10. The flakes of claim 5 in which the embossed flakes have a diffraction grating pattern of less than 14,000 grooves per centimeter, and a groove depth of less than 140 nm.

11. A multi-layer laminate having a decorative layer with a print pattern made from a coating or printing ink containing the embossed flakes of anyone of claims 5 to 10, in which the decorative print pattern is optionally applied to a pigmented opaque base coat applied to a polymeric substrate sheet, and in which the laminate is thermoformable to a three-dimensional shape without degrading reflective optical properties of the decorative print pattern.

12. A resinous coating containing the embossed flakes of anyone of claims 5 to 10, that produce a combined sparkle or glitter effect with color shift across the color spectrum, and in which the embossed flakes optionally have an average D50 particle size at or above 100 microns.

## Patentansprüche

1. Verfahren zur Herstellung von geprägten feinen, teilchenförmigen, dünnen, metallischen Plättchen mit Glanz und Farbintensität, umfassend das Bereitstellen einer Ablöseoberfläche (12) auf einem Träger (10), das Prägen der Ablöseoberfläche (12) mit einem Beugungsgittermuster (14, 16, 18), das ein Winkellinienmuster von größer als 45° aufweist, das Metallisieren der geprägten Ablöseoberfläche (12) mit einem dünnen reflektierenden Metallfilm, das Entfernen des Metallfilms von der Ablöseoberfläche (12), um eine Lösungsmitteldispersion der geprägten Metallplättchen zu bilden, welche das Beugungsgittermuster replizieren, und das Kontrollieren der Teilchengröße der in der Dispersion enthaltenen Plättchen, um die darin enthaltenen geprägten Plättchen bei einer Teilchengröße von D50 von bei oder über 75 µm beizubehalten.

2. Verfahren zum Bilden einer ersten Beschichtung, enthaltend die geprägten Plättchen gemäß Anspruch 1, dispergiert in einem polymeren Bindemittel, worin die erste Beschichtung einen höheren Chromatizitätsstand und einen höheren Farbintensitätsstand bei 75° und 110° Winkelmessung, wenn an einem Multi-Winkelspectrophotometer gemessen, aufweist, wenn verglichen mit einer zweiten Beschichtung, enthaltend eine Dispersion von D50-geprägten Plättchen mit einer Größe von 50 µm, hergestellt durch ein ähnliches Verfahren und enthalten in dem gleichen polymeren Bindemittel.

3. Verfahren gemäß Anspruch 1, worin die geprägten Plättchen ein Beugungsgittermuster von weniger als 14.000 Kerben bzw. Wellen pro Zentimeter, eine Plättchendicke von 50 nm bis 100 nm und eine Rillentiefe von 125 nm bis 140 nm aufweisen.

4. Verfahren gemäß Anspruch 1, worin die in der Lösungsmitteldispersion enthaltenen metallischen Plättchen keiner angelegten Energie unterworfen werden, welche die Teilchengröße von größer als Mischen mit niedriger Geschwindigkeit reduzieren würde oder welche die Teilchengröße mehr als 20 µm reduzieren würde.

5. Reflektierende Metallplättchen, die durch Replizieren eines Beugungsgittermusters mit einem Monolinienprägewinkel über 45° geprägt worden sind, wobei die Teilchen eine durchschnittliche Teilchengröße D50 bei oder oberhalb 75 µm und eine Plättchendicke von 50 nm bis 100 nm aufweisen.

6. Plättchen gemäß Anspruch 5, wobei die geprägten Plättchen einen Teilchengrößenbereich von (a) oder (b) aufweisen:
(a) von 75 bis 200 µm oder
(b) von 75 bis 150 µm, und
wobei die metallischen Plättchen einen Dickenbereich von (c) oder (d) aufweisen:
(c) von 5 nm bis 100 nm oder
(d) von 50 nm bis 100 nm, oder alternativ eine optische Dichte von 1,0 bis 3,5.

7. Plättchen gemäß Anspruch 5, wobei das Beugungsgittermuster von 5.000 bis weniger als 14.000 Rillen pro cm aufweist.

8. Plättchen gemäß Anspruch 5, wobei die geprägten Plättchen in einer Trokkenfilmbeschichtung mit einer größeren gemessenen Chromatizität und Farbdichte bei 75° und 110° (gemessen über ein Multi-Winkel-Spektrophotometer) aufweisen, wenn verglichen mit einer ähnlichen Beschichtung, enthaltend Plättchen, geprägt mit einem Beugungsgittermuster bei 45° und mit einer Teilchengröße von 50 µm.

9. Plättchen gemäß Anspruch 5, wobei die geprägten Plättchen eine optische Dichte von 3,0 oder mehr und eine durchschnittliche Plättchengröße D50 von größer als 200 µm aufweisen.

10. Plättchen gemäß Anspruch 5, wobei die geprägten Plättchen ein Beugungsgittermuster von weniger als 14.000 Rillen pro cm und eine Rillentiefe von weniger als 140 nm aufweisen.

11. Mehrschichtlaminat, das eine dekorative Schicht mit einem Druckmuster aufweist, hergestellt aus einer Beschichtung oder einer Drucktinte, enthaltend die geprägten Plättchen gemäß einem der Ansprüche 5 bis 10, wobei das Muster des dekorativen Drucks gegebenenfalls auf einer pigmentierten, opaken Basisbeschichtung, aufgebracht auf ein polymeres Substratblatt, angeordnet ist, und wobei das Laminat zu einer dreidimensionalen Form, ohne Herabsetzen der reflektierenden optischen Eigenschaften des dekorativen Druckmusters, thermoformbar ist.

12. Harzförmige Beschichtung, enthaltend die geprägten Plättchen gemäß einem der Ansprüche 5 bis 10, welche einen kombinierten Funkel- oder Glitzereffekt mit einem Farbshift entlang des Farbspektrums liefern, und wobei die geprägten Plättchen gegebenenfalls eine durchschnittliche Teilchengröße D50 bei oder oberhalb 100 µm aufweisen.

## Revendications

1. Procédé de fabrication de fins flocons métalliques cannelés sous forme de particules fines possédant une brillance et une intensité de couleur, comprenant la fourniture d'une surface de libération (12) sur un support (10), le cannelage de la surface de libération (12) avec un motif de réseau de diffraction (14, 16, 18) ayant un motif angulaire de ligne supérieur à 45°, la métallisation de la surface de libération cannelée (12) avec un film métallique réfléchissant fin, le retrait du film métallique de la surface de libération (12) pour former une dispersion, dans un solvant, des flocons métalliques cannelés présentant le motif de réseau de diffraction répliqué, et le contrôle de la taille de particule des flocons contenus dans la dispersion pour que la taille de particule D50 des flocons cannelés contenus dedans soit maintenue à une valeur supérieure ou égale à 75 microns.

2. Procédé de formation d'un premier revêtement contenant les flocons cannelés selon la revendication 1 dispersés dans un liant polymère, dans lequel le premier revêtement présente une lecture de chromaticité plus élevée et une lecture d'intensité de couleur plus élevée à des mesures angulaires de 75° et 110°, quand la mesure est réalisée avec un spectrophotomètre à angles multiples, par rapport à un second revêtement contenant une dispersion de flocons cannelés ayant une taille D50 de 50 microns préparés par un procédé similaire et contenus dans le même liant polymère.

3. Procédé selon la revendication 1, dans lequel les flocons cannelés possèdent un motif de réseau de diffraction inférieur à 14 000 cannelures par centimètre, une épaisseur de flocon de 50 nm à 100 nm, et une profondeur de cannelure de 125 nm à 140 nm.

4. Procédé selon la revendication 1, dans lequel les flocons métalliques contenus dans la dispersion de solvant ne sont soumis à aucune énergie appliquée qui réduirait la taille des particules supérieure à un mélange à basse vitesse, ou qui réduirait la taille des particules de plus de 20 microns.

5. Flocons métalliques réfléchissants qui ont été cannelés en répliquant un motif de réseau de diffraction présentant un angle de cannelage par ligne supérieur à 45°, les particules possédant une taille moyenne de particule D50 supérieure ou égale à 75 microns et une épaisseur de flocon de 50 nm à 100 nm.

6. Flocons selon la revendication 5, dans lesquels la plage de tailles de particule des flocons cannelés est
(a) ou (b) :
(a) de 75 microns à 200 microns, ou
(b) de 75 microns à 150 microns ; et
dans lesquels la plage d'épaisseur des flocons métalliques est (c) ou (d) :
(c) de 5 nm à 100 nm ; ou
(d) de 50 nm à 100 nm ; ou en variante, une densité optique de 1,0 à 3,5.

7. Flocons selon la revendication 5, dans lesquels le motif de réseau de diffraction possède de 5 000 à moins de 14 000 rainures par cm.

8. Flocons selon la revendication 5, dans lesquels les flocons cannelés sont contenus dans un revêtement de film sec dont la lecture de chromaticité et la lecture d'intensité de couleur mesurées à 75° et 110° sont plus élevées (quand la mesure est réalisée avec un spectrophotomètre à angles multiples) par rapport à un revêtement similaire contenant des flocons cannelés à un motif de réseau de diffraction de 45° et possédant une taille de particule de 50 microns.

9. Flocons selon la revendication 5, dans lesquels les flocons cannelés possèdent une densité optique de 3,0 ou plus et une taille moyenne D50 de flocons supérieure à 200 microns.

10. Flocons selon la revendication 5, dans lesquels les flocons cannelés possèdent un motif de réseau de diffraction inférieur à 14 000 cannelures par centimètre et une profondeur de cannelure inférieure à 140 nm.

11. Stratifié multicouche possédant une couche décorative ayant un motif imprimé préparé à partir d'un revêtement ou d'une encre d'impression contenant les flocons cannelés selon l'une quelconque des revendications 5 à 10, dans lequel le motif d'impression décoratif est facultativement appliqué sur un revêtement de base opaque pigmenté appliqué sur une feuille de substrat polymère, et dans lequel le stratifié peut être thermoformé sous une forme tridimensionnelle sans dégrader les propriétés optiques de réflexion du motif d'impression décoratif.

12. Revêtement résineux contenant les flocons cannelés selon l'une quelconque des revendications 5 à 10, produisant un effet combiné de brillance ou de scintillement avec une variation chromatique traversant le spectre des couleurs, et dans lequel les flocons cannelés possèdent facultativement une taille moyenne de particule D50 supérieure ou égale à 100 microns.
